# EUROPEAN PATENT SPECIFICATION

(11) **EP 2 185 132 B1**
(45) Date of publication and mention of the grant of the patent: **17.02.2016**
(21) Application number: 07874236.8
(22) Date of filing: 26.10.2007
(51) Int. Cl.: A61L 31/06, A61K 9/50, C08G 79/02, C08J 3/12, C09D 185/02

(54) **COLOR-CODED AND SIZED LOADABLE POLYMERIC PARTICLES FOR THERAPEUTIC AND/OR DIAGNOSTIC APPLICATIONS AND METHODS OF PREPARING AND USING THE SAME**
FARBIG CODIERTE UND GRUNDIERTE LADBARE POLYMERTEILCHEN FÜR THERAPEUTISCHE UND/ODER DIAGNOSTISCHE ANWENDUNGEN UND VERFAHREN ZU IHRER HERSTELLUNG UND VERWENDUNG
PARTICULES POLYMÉRIQUES CHARGEABLES À CODAGE ET TAILLE DE COULEUR POUR APPLICATIONS THÉRAPEUTIQUES ET/OU DIAGNOSTIQUES, ET PROCÉDÉS DE PRÉPARATION ET D'UTILISATION DE CES PARTICULES

(30) Priority: 25.07.2007 US 962015 P
(43) Date of publication of application: 19.05.2010
(60) Divisional application: 12188376.3
(73) Proprietor: CeloNova Biosciences, Inc., Peachtree City, GA 30269 (US)
(72) Inventor: FRITZ, Ulf, 69434 Hirschhorn (DE); FRITZ, Olaf, 69434 Hirschhorn (DE); GORDY, Thomas, A., Newnan, GA 30265 (US); WOJCIK, Ronald, Canton, GA 30114 (US); BLUMMEL, Jacques, D-68229 Mannheim (DE); KULLER, Alexander, D-69123 Heidelberg (DE)
(74) Representative: Instone, Alicia Claire
(86) International application number: PCT/US2007/082659
(87) International publication number: WO 2009/014549

(56) References cited:
- US-A1- 2003 099 683
- US-A1- 2006 088 476
- SANGAMESH G KUMBAR ET AL: "In Vitro and In Vivo Characterization of Biodegradable Poly(organophosphazenes) for Biomedical Applications" 30 December 2006 (2006-12-30), JOURNAL OF INORGANIC AND ORGANOMETALLIC POLYMERS, KLUWER ACADEMIC PUBLISHERS-PLENUM PUBLISHERS, NE, PAGE(S) 365 - 385 , XP019481702 ISSN: 1574-1451 abstract figure 1
- CALICETI PAOLO ET AL: "Polyphosphazene microspheres for insulin delivery" INTERNATIONAL JOURNAL OF PHARMACEUTICS, ELSEVIER BV, NL, vol. 211, no. 1-2, 15 December 2000 (2000-12-15), pages 57-65, XP002383273 ISSN: 0378-5173

## Description

### BACKGROUND OF THE INVENTION

Small particles, including microspheres and nanospheres, have many medical uses in diagnostic and therapeutic procedures. In selected clinical applications, it may be advantageous to provide specific sizes of such microspheres and nanospheres to a user. Such sizing of microspheres and nanospheres may allow for selective embolization of certain sized blood vessels in specific clinical uses. It may further be advantageous to provide a user with color-coded microspheres or nanospheres to allow ready identification of the sized particles in use. Such color-coded microspheres or nanospheres may further be provided in like color- coded delivery or containment devices to enhance user identification and provide visual confirmation of the use of a specifically desired size of microspheres or nanospheres.

Most prior art particles used in medical applications are characterized by numerous disadvantages including irritation of the tissues with which they come in contact and initiation of adverse immune reactions. Additionally, many of the materials used to prepare the prior art particles may degrade relatively rapidly within the mammalian body, thereby detracting from their utility in certain procedures where long term presence of intact particles may be necessary. Moreover, the degradation of the prior art materials may release toxic or irritating compounds causing adverse reactions in the patients.

It is also a problem in the art for certain types of prior art particles that it is difficult to achieve desirable suspension properties when the particles are incorporated into a delivery suspension for injection into a site in the body to be treated. Many times, the particles settle out or tend to "float" in the solution such that they are not uniformly suspended for even delivery. Furthermore, particles may tend to aggregate within the delivery solution and/or adhere to some part of the delivery device, making it necessary to compensate for these adhesive/attractive forces.

In order to achieve a stable dispersion, it is known to add suitable dispersing agents that may include surfactants directed at breaking down attractive particle interaction. Depending on the nature of the particle interaction, the following materials may be used: cationic, anionic or nonionic surfactants such as Tween™ 20, Tween™ 40, Tween™ 80, polyethylene glycols, sodium dodecyl sulfate, various naturally occurring proteins such as serum albumin, or any other macromolecular surfactants in the delivery formulation. Furthermore thickening agents can be used help prevent particles from settling by sedimentation and to increase solution viscosity, for example, polyvinyl alcohols, polyvinyl pyrrolidones, sugars or dextrins. Density additives may also be used to achieve buoyancy.

It can also be difficult to visualize microparticles in solution to determine their degree of suspension when using clear, transparent polymeric acrylate hydrogel beads in aqueous suspension. Attempts to use the inert precipitate, barium sulfate, in particle form is known as an additive for bone cement, for silicones for rendering items visible during X-ray examination and for providing radiopacity to polymeric acrylate particles. See Jayakrishnan et al., Bull. Mat. Sci., Vol. 12, No. 1, pp. 17-25 (1989). The barium sulfate also is known for improving fluidization, and is often used as an inorganic filler to impart anti-stick behavior to moist, aggregated particles. Other prior art attempts to increase visualization of microparticles include use of gold, for example, Embosphere Gold™ provides a magenta color to acrylate microparticles using small amounts of gold.

In certain medical applications, it may further be of value to provide microparticles such as microspheres in one or more sizes. Furthermore, it may also be of value to a user to provide each of such sizes of microspheres incorporated with color-coded associated dyes to indicate the microsphere size to the user. In yet other applications of use, it may further be of value to provide sized and color-coded microspheres to a user in similarly color-coded syringes or other containers for transport and delivery to further aid a user in identifying the size of microspheres being used.

There thus exists in the art a need for small particles that can be formed to have a preferential generally spherical configuration for certain applications such as various therapeutic and diagnostic procedures which are not degraded by the natural systems of the mammalian system, are biocompatible, are easy to visualize in suspension while in use and/or demonstrate acceptable physical and suspension properties.

US2006/0088476 describes particles for use in therapeutic and/or diagnostic procedures. The particles include poly[bis(trifluoroethoxy) phosphazene] and/or a derivatives thereof which may be present throughout the particles or within an outer coating of the particles. The particles can also include a core having a hydrogel formed from an acrylic-based polymer. Barium sulfate may also be provided to the core of the particles as a coating or absorbed within the core of the particles. The particles can be used to minimize blood flow to mammalian tissues by occluding at least a portion of a blood vessel of the mammal, or to deliver an active agent to a localized area within a body of a mammal by contacting a localized area with at least one of the particles.; Further, the particles are useful in sustained release formulations including active agent(s) for oral administration, as tracer particles for injection into the bloodstream of a mammal or for use in enhanced ultrasound imaging. The particles may include agents for increasing density for achieving useful buoyancy levels in suspension.

### BRIEF SUMMARY OF THE INVENTION

According to a first aspect of the present invention there is provided color-coded polymeric particles according to claims 1 to 9. According to a second aspect of the invention there is provided a method for making color-coded polymeric particles according to claims 10 to 14. Also described herein is a particle for use in a therapeutic and/or diagnostic procedure. The particle comprises poly[bis(trifluoroethoxy) phosphazene] and/or a derivative thereof.

Also described herein are particles comprising poly[bis(trifluoroethoxy) phosphazene and/or a derivative thereof provided as microspheres provided in one or more specified sizes.

Also described herein are particles comprising poly[bis(trifluoroethoxy) phosphazene and/or a derivative thereof provided as sized microspheres and further comprising a color-coded dye incorporated into or attached to the exterior of the microspheres to visually aid a user in identifying the size of microspheres in use.

Microspheres described herein may further be provided as sized microspheres further comprising a color-coded dye incorporated into or attached to the exterior of the microspheres and contained or delivered in a similarly color-coded syringe or other transport or delivery container to further visually aid a user in providing a visual confirmation of the specific size of microspheres in use.

Also described herein is a method of minimizing blood flow to a tissue in a mammal comprising occluding at least a portion of a blood vessel of the mammal with at least one particle, wherein the particle comprises a poly[bis(trifluoroethoxy) phosphazene] and/or a derivative thereof.

Further described herein is a method of delivering an active agent to a localized area within a body of a mammal comprising contacting the localized area with at least one of a particle comprising poly[bis(trifluoroethoxy) phosphazene] and/or a derivative thereof and an active agent, such that an effective amount of the active agent is exposed to the localized area.

Also described herein is a sustained release formulation of an active agent for oral administration, the formulation comprising a polymer capsule and an active agent, wherein the polymeric capsule comprises poly[bis(trifluoroethoxy) phosphazene] and/or a derivative thereof.

Also described herein is a method of tracing the passage of a particle through a blood vessel in a mammal, the method comprising injecting into the bloodstream of a mammal at least one tracer particle, the tracer particle comprising poly[bis(trifluoroethoxy) phosphazene] and/or a derivative thereof and a contrast agent, and imaging the route of the particle.

Additionally, a method of enhanced ultrasound imaging is described herein. The method comprises administering to an ultrasound subject at least one hollow microcapsule comprising poly[bis(trifluoroethoxy) phosphazene] and/or a derivative thereof to an area of the ultrasound subject, and imaging the area of the subject using ultrasound.

Also described herein is a method of delivering an active agent to a localized area within the body of a mammal comprising contacting the localized area with at least one of a particle comprising poly[bis(trifluoroethoxy) phosphazene] and/or a derivative thereof and an active agent, such that an effective amount of the active agent is exposed to the localized area, wherein the particle comprises an agent to increase density.

Further, a method for minimizing agglomeration of particles formed from acrylic- based polymers is described in which the method comprises providing barium sulfate to the core and/or surface of the particles.

### BRIEF DESCRIPTION OF THE SEVERAL VIEWS OF THE DRAWINGS

The foregoing summary, as well as the following detailed description of the invention, will be better understood when read in conjunction with the appended drawings. For the purpose of illustrating the invention, there are shown in the drawings embodiments that are presently preferred. It should be understood, however, that the invention is not limited to the precise arrangements and instrumentalities shown.

In the drawings:
Fig. 1 shows a schematic representation of a general cryoextraction scheme used to prepare particles according to one embodiment of the invention;
Fig. 2 shows the manual dripping technique by which the polymer solution was supplied to liquid nitrogen in preparation of the microspheres of Example 1, herein;
Fig. 3A shows representative different sized and color-coded microspheres A, B, and C of the present invention.
Fig. 3B shows a cross-sectional drawing of a conceptual blood vessel with arrows indicating the direction of blood flow, wherein the blood vessel tapers from a larger proximal diameter to a smaller distal diameter, and wherein different sized and color-coded microspheres of the present invention have been sequentially injected in order of ascending size to occlude the vessel.
Fig. 3C shows a syringe containing microspheres of the present invention wherein the microspheres are sized and color-coded to indicate their size, and wherein the syringe is further similarly-color coded to facilitate user identification and verification of the sized microspheres in use.

### DETAILED DESCRIPTION OF THE INVENTION

Described herein are particles that may be manufactured using poly[bis(trifluoroethoxy) phosphazene] and/or derivatives thereof, as well as methods of preparing such particles. Additionally, described herein are therapeutic and/or diagnostic methods and procedures which use the particles as described herein, including methods of embolization using the particles, methods of delivery of an active agent using the particle (either orally or locally), methods of tracing or visualizing blood or other biological fluids through the body using the particles, and methods of enhanced ultrasound (sonography) using the particles.

Also included are sustained release drug delivery formulations for oral administration including the particles for localized delivery of an active agent to the gastrointestinal system and/or systemic delivery of an active agent as well as a sustained release drug delivery formulation that can be injected subcutaneously or intravenously for localized delivery of an active agent.

All of the methods, compositions and formulations of the invention utilize at least one particle as described herein. "Particle" and "particles" as used herein mean a substantially spherical or ellipsoid article(s), hollow or solid, that may have any diameter suitable for use in the specific methods and applications described below, including a microsphere(s) and a nanosphere(s), beads and other bodies of a similar nature known in the art.

The shell of the preferred particles of the invention according to one embodiment described herein are composed, in whole or in part, of the specific polyphosphazene polymer known as poly[bis(trifluoroethoxy) phosphazene] or a derivative of poly[bis(trifluoroethoxy) phosphazene]. Use of this specific polymer provides particles that are at least in part inorganic in that they include an inorganic polymer backbone and which are also biocompatible in that when introduced into a mammal (including humans and animals), they do not significantly induce a response of the specific or non-specific immune systems. The particles may be used as controlled drug delivery vehicles or tracer particles for the visualization of blood vessels and other organs.

The particles are useful in a variety of therapeutic and/or diagnostic procedures in part because they can be prepared in sizes large enough to occlude a blood vessel as well as small enough to easily pass through the smaller vessels, e.g., visualization or drug delivery purposes. Additionally, owing to the biocompatible nature of the polymer, the particles facilitate avoidance or elimination of immunogenic reactions generally encountered when foreign bodies are introduced into a mammalian body, such as "implant rejection" or "allergic shock," and other adverse reactions of the immune system. Moreover, it has been found that the particles of the invention exhibit reduced biodegradation in vivo, thereby increasing the long-term stability of the particle in the biological environment. Moreover, in those situations where some degradation is undergone by the polymer in the particle, the products released from the degradation include only non-toxic concentrations of phosphorous, ammonia, and trifluoroethanol, which, advantageously, is known to promote anti-inflammatory responses when in contact with mammalian tissue.

The shell of each particle in the invention is formed at least in part of the polymer, poly[bis{2,2,2-trifluoroethoxy) phosphazene] or a derivative thereof (referred to further herein as "poly[bis(trifluoroethoxy)phosphazene]". As described herein, the polymer poly[bis(2,2,2-trifluoroethoxy)phosphazene] or derivatives thereof have chemical and biological qualities that distinguish this polymer from other know polymers in general, and from other know polyphosphazenes in particular. In one aspect of this invention, the polyphosphazene is poly[bis(2,2,2-trifluoroethoxy) phosphazene] or derivatives thereof, such as other alkoxide, halogenated alkoxide, or fluorinated alkoxide substituted analogs thereof. The preferred poly[bis(trifluoroethoxy)phosphazene] polymer is made up of repeating monomers represented by the formula (I) shown below: 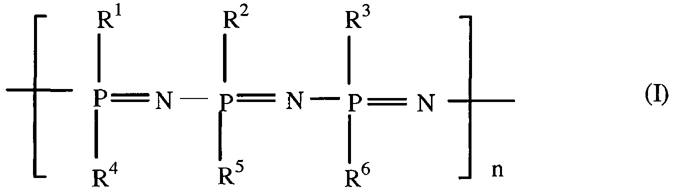 wherein R¹ to R⁶ are all trifluoroethoxy (OCH₂CF₃) groups, and wherein n may vary from at least about 40 to about 100,000, as disclosed herein. Alternatively, one may use derivatives of this polymer in the present invention. The term "derivative" or "derivatives" is meant to refer to polymers made up of monomers having the structure of formula I but where one or more of the R¹ to R⁶ functional group(s) is replaced by a different functional group(s), such as an unsubstituted alkoxide, a halogenated alkoxide, a fluorinated alkoxide, or any combination thereof, or where one or more of the R¹ to R⁶ is replaced by any of the other functional group(s) disclosed herein, but where the biological inertness of the polymer is not substantially altered.

In one aspect of the polyphosphazene of formula (I) illustrated above, for example, at least one of the substituents R¹ to R⁶ can be an unsubstituted alkoxy substituent, such as methoxy (OCH₃), ethoxy (OCH₂CH₃) or n-propoxy (OCH₂CH₂CH₃). In another aspect, for example, at least one of the substituents R¹ to R⁶ is an alkoxy group substituted with at least one fluorine atom. Examples of useful fluorine-substituted alkoxy groups R¹ to R⁶ include, but are not limited to OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)sCF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, and the like. Thus, while trifluoroethoxy (OCH₂CF₃) groups are preferred, these further exemplary functional groups also may be used alone, in combination with trifluoroethoxy, or in combination with each other. In one aspect, examples of especially useful fluorinated alkoxide functional groups that may be used include, but are not limited to, 2,2,3,3,3-pentafluoropropyloxy (OCH₂CF₂CF₃), 2,2,2,2'2'2'-hexafluoroisopropyloxy (OCH(CF₃)₂), 2,2,3,3,4,4,4-heptafluorobutyloxy (OCH₂CF₂CF₂CF₃), 3,3,4,4,5,5,6,6,7,7,8,8,8-tridecafluorooctyloxy (OCH₂(CF₂)₇CF₃), 2,2,3,3,-tetrafluoropropyloxy (OCH₂CF₂CHF₂), 2,2,3,3,4,4-hexafluorobutyloxy (OCH₂CF₂CF₂CHF₂), 3,3,4,4,5,5,6,6,7,7, 8,8-dodecafluorooctyloxy (OCH₂(CF₂)₇CHF₂), and the like, including combinations thereof.

Further, in some embodiments, 1% or less of the R¹ to R⁶ groups may be alkenoxy groups, a feature that may assist in crosslinking to provide a more elastomeric phosphazene polymer. In this aspect, alkenoxy groups include OCH₂CH=CH₂, OCH₂CH₂CH=CH₂, allylphenoxy groups, and the like, including combinations thereof. Also in formula (I) illustrated herein, the residues R¹ to R⁶ are each independently variable and therefore can be the same or different.

By indicating that n can be as large as ∞ in formula I, it is intended to specify values of n that encompass polyphosphazene polymers that can have an average molecular weight of up to about 75 million Daltons. For example, in one aspect, n can vary from at least about 40 to about 100,000. In another aspect, by indicating that n can be as large as ∞ in formula I, it is intended to specify values of n from about 4,000 to about 50,000, more preferably, n is about 7,000 to about 40,000 and most preferably n is about 13,000 to about 30,000.

In another aspect of this invention, the polymer used to prepare the polymers disclosed herein has a molecular weight based on the above formula, which can be a molecular weight of at least about 70,000 g/mol, more preferably at least about 1,000,000 g/mol, and still more preferably a molecular weight of at least about 3x10⁶ g/mol to about 20x10⁶ g/mol. Most preferred are polymers having molecular weights of at least about 10,000,000 g/mol.

In a further aspect of the polyphosphazene formula (1) illustrated herein, n is 2 to ∞, and R¹ to R⁶ are groups which are each selected independently from alkyl, aminoalkyl, haloalkyl, thioalkyl, thioaryl, alkoxy, haloalkoxy, aryloxy, haloaryloxy, alkylthiolate, arylthiolate. alkylsulphonyl, alkylamino, dialkylamino, heterocycloalkyl comprising one or more heteroatoms selected from nitrogen, oxygen, sulfur, phosphorus, or a combination thereof, or heteroaryl comprising one or more heteroatoms selected from nitrogen, oxygen, sulfur, phosphorus, or a combination thereof. In this aspect of formula (I), the pendant side groups or moieties (also termed "residues") R¹ to R⁶ are each independently variable and therefore can be the same or different. Further, R¹ to R⁶ can be substituted or unsubstituted. The alkyl groups or moieties within the alkoxy, alkylsulphonyl, dialkylamino, and other alkyl- containing groups can be, for example, straight or branched chain alkyl groups having from 1 to 20 carbon atoms, typically from 1 to 12 carbon atoms, it being possible for the alkyl groups to be further substituted, for example, by at least one halogen atom, such as a fluorine atom or other functional group such as those noted for the R¹ to R⁶ groups above. By specifying alkyl groups such as propyl or butyl, it is intended to encompass any isomer of the particular alkyl group.

In one aspect, examples of alkoxy groups include methoxy, ethoxy, propoxy, and butoxy groups, and the like, which can also be further substituted. For example the alkoxy group can be substituted by at least one fluorine atom, with 2,2,2- trifluoroethoxy constituting a useful alkoxy group. In another aspect, one or more of the alkoxy groups contains at least one fluorine atom. Further, the alkoxy group can contain at least two fluorine atoms or the alkoxy group can contain three fluorine atoms. For example, the polyphosphazene that is combined with the silicone can be poly[bis(2,2,2- trifluoroethoxy)phosphazene]. Alkoxy groups of the polymer can also be combinations of the aforementioned embodiments wherein one or more fluorine atoms are present on the polyphosphazene in combination with other groups or atoms,

Examples of alkylsulphonyl substituents include methylsulphonyl, ethylsulphonyl, propylsulphonyl, and butylsulphonyl groups. Examples of dialkylamino substituents include, but are not limited to, dimethyl-, diethyl-, dipropyl and dibutylamino groups. Again, by specifying alkyl groups such as propyl or butyl, it is intended to encompass any isomer of the particular alkyl group,

Exemplary aryloxy groups include, for example, compounds having one or more aromatic ring systems having at least one oxygen atom, non-oxygenated atom, and/or rings having alkoxy substituents, it being possible for the aryl group to be substituted for example by at least one alkyl or alkoxy substituent defined above. Examples of aryloxy groups include phenoxy and naphthoxy groups, and derivatives thereof including, for example, substituted phenoxy and naphthoxy groups.

The heterocycloalkyl group can be. for example, a ring system which contains from 3 to 10 atoms, at least one ring atom being a nitrogen, oxygen, sulfur, phosphorus, or any combination of these heteroatoms. The hetereocycloalkyl group can be substituted, for example, by at least one alkyl or alkoxy substituent as defined above. Examples of heterocycloalkyl groups include piperidinyl, piperazinyl, pyrrolidinyl, and morpholinyl groups, and substituted analogs thereof.

The heteroaryl group can be, for example, a compound having one or more aromatic ring systems, at least one ring atom being a nitrogen, an oxygen, a sulfur, a phosphorus, or any combination of these heteroatoms. The heteroaryl group can be substituted for example by at least one alkyl or alkoxy substituent defined above. Examples of heteroaryl groups include imidazolyl, thiophene, furane, oxazolyl, pyrrolyl, pyridinyl, pyridinolyl, isoquinolinyl, and quinolinyl groups, and derivatives thereof, such as substituted groups.

The diameter of a particle formed according to the invention will vary depending on the end application in which the particle is to be used. The diameter of such particles is preferably about 1 to about 5,000 µm, with a diameter of about 1 to about 1.000 µm being most preferred. Other preferred sizes include diameters of about 200 to about 500 µm, about 1 to about 200 µm and greater than about 500 µm. In methods using the particle where more than one particle is preferred it is not necessary that all particles are of the same diameter or shape. In one aspect, the polymeric particles are substantially uniform in size, meaning that size of the particles can be determined by the process by which they are prepared and isolated, and they are characterized by a narrow size distribution. By substantially uniform in size, it is generally intended to reflect that the particle size according to the design specification may vary less than or equal to about ±5%, less than or equal to about ±10%, less than or equal to about ±15%, less than or equal to about ±20%, less than or equal to about ±25%, less than or equal to about ±30%, or less than or equal to about ±35% from the design specification. In one aspect, for example, size distributions of the particles disclosed herein may become more narrow as the design specification of the particle to be fabricated becomes larger. For example, particles between about 700 µm and about 1000 µm may vary less than or equal to only about ±3-5% from the design specification, whereas particles between about 40 µm and about 100 µm may vary less than or equal to about ±20-25% from the design specification.

The particles may also include other compounds which function to enhance, alter or otherwise modify the behavior of the polymer or particle either during its preparation or in its therapeutic and/or diagnostic use. For example, active agents such as peptides, proteins, hormones, carbohydrates, polysaccharides, nucleic acids, lipids, vitamins, steroids and organic or inorganic drugs may be incorporated into the particle. Excipients such as dextran, other sugars, polyethylene glycol, glucose, and various salts, including, for example, chitosan glutamate, may be included in the particle.

Additionally, if desired, polymers other than the poly[bis(trifluoroethoxy) phosphazene] and/or its derivative may be included with in the particle. Examples of polymers may include poly(lactic acid), poly(lactic-co-glycolic acid), poly(caprolactone), polycarbonates, polyamides, polyanhydrides, polyamino acids, polyorthoesters, polyacetals, polycyanoacrylates, and polyurethanes. Other polymers include polyacrylates, ethylene-vinyl acetate co-polymers, acyl substituted cellulose acetates and derivatives thereof, degradable or non-degradable polyurethanes, polystyrenes, polyvinylchloride, polyvinyl fluoride, poly(vinyl imidazole), chlorosulphonated polyolefins, and polyethylene oxide. Examples of polyacrylates include acrylic acid, butyl acrylate, ethylhexyl acrylate, methyl acrylate, ethyl acrylate, acrylonitrile, methyl methacrylate, TMPTA (trimethylolpropane triacrylate), and the like. One may incorporate the selected compounds by any means known in the art, including diffusing, inserting or entrapping the additional compounds in the matrix of an already formed particle or by adding the additional compound to a polymer melt or to a polymer solvent in the preparation of the particle such as described herein.

The loaded or unloaded particle may be coated with an additional polymer layer or layers, including polymers such as those mentioned hereinabove. PTFEP or its derivatives are used to form a coating on a particle formed of polymers or copolymers that are used to form particles as described herein. PTFEP is applied as a coating on a microparticle(s) formed of an animic acrylic-based polymer as set forth in further detail below.

Coatings are beneficial, for example, if the particle(s) are to be used in a sustained release, orally administered, drug delivery formulation (enteric coating) or if the particles are to be loaded with a potentially toxic contrast agent (non-biodegradable coating).

The microspheres may be prepared by any means known in the art that is suitable for the preparation of particles containing poly[bis(trifluoroethoxy) phosphazene]. In a procedure according to an embodiment herein a "polymer solution" is prepared by mixing one or more polymer solvent(s) and the PTFEP and/or a derivative thereof until the polymer is dissolved,

Suitable solvents for use in the preparation of the polymer solution include any in which the polymer PTFEP and/or its derivatives are soluble. Exemplary solvents include, without limitation, ethyl-, propyl-, butyl-, pentyl-, octylacetate, acetone, methylethylketone, methylpropylketone, methylisobutylketone, tetrahydrofurane, cyclohexanone, dimethylacetamide, acetonitrile, dimethyl ether, hexafluorobenzene or combinations thereof.

The polymer solution contains the PTFEP and/or its derivative polymer in a concentration of about 1% by weight of polymer to 20% by weight of polymer, preferably about 5% to 10% by weight of polymer. Other polymers, as discussed above, may be present in the solution, or may be added to the vessel in the form of a second solution powder or other form, if one wishes to include such polymers in the final particle.

In carrying out the process, the polymer solution is next dispensed, preferably in the form of drops or an aerosol, into a vessel containing a non-solvent. By "non-solvent" it is meant any organic or inorganic solvents that do not substantially dissolve the PTFEP polymer and which have a melting point that is lower relative to the melting point of the solvent in which the polymer is dissolved ("polymer solvent"), so that the non-solvent thaws before the solvent thaws in the course of the incubation step. Preferably, this difference between the melting point of the non-solvent and the polymer solvent is about 10° C, more preferably about 15° C, and most preferably, greater than about 20° C. Under certain conditions it has been found that the structural integrity of the resultant particle may be enhanced if the difference of the melting points of the polymer solvent and of the non-solvent is greater than 15° C. However, it is sufficient that the non-solvent point is merely slightly lower than that of the polymer solvent.

The non-solvent/polymer solvent combination is incubated for approximately 1 to 5 days or until the polymer solvent has been completely removed from the particles. While not wishing to be bound by theory, it is hypothesized that during the incubation, the non-solvent functions to extract the polymer solvent from the microscopic polymer solution droplets from the particles such that the polymer is at least gelled. As the incubation period passes, the droplets will shrink and the solvent becomes further extracted, leading to a hardened outer polymeric shell containing a gelled polymer core, and finally, after completion of the incubation, a complete removal of the residual solvent. To ensure that the polymeric droplets retain a substantially spherical shape during the incubation period, they are maintained in a frozen or substantially gelled state during most if not all of the incubation period. Therefore, the non-solvent temperature may stay below the melting point of the solvent during the cryoextraction process,

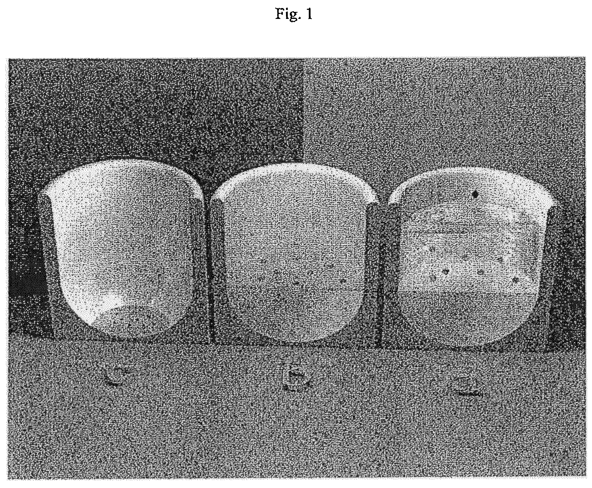

As shown in Fig. 1, at the vessel labeled (a), polymer solution droplets are shown being dispensed either with a syringe or other device at a controlled rate onto a top layer of liquid nitrogen. The nitrogen layer is situated over a bottom layer consisting of the selected non-solvent, which will eventually serve to extract the solvent from the frozen polymer solution droplets. The non-solvent layer has been previously frozen with liquid nitrogen prior to the dispensing of the polymer solution. The vessel labeled (b) shows the onset of the dewing of the frozen nonsolvent, into which the frozen polymeric droplets will sink. The vessel labeled (c) shows the cryoextraction procedure after approximately three days of incubation wherein the polymer solution droplets, incubated within the non-solvent, have been depleted of a substantial amount of solvent. The result is a gelled, polymeric particle in the form of a bead having a hardened outer shell. As can be seen by the representation, the non-solvent height within the vessel is slightly reduced due to some evaporation of the non-solvent. The size of the beads will shrink quite substantially during this process depending on the initial concentration of the polymer in the polymer solution.

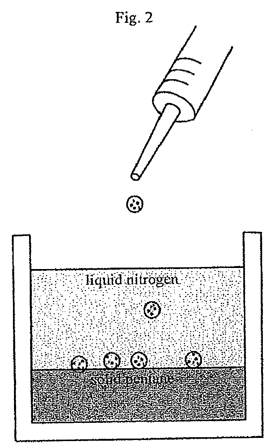

In one embodiment of a method of preparing a PTFEP-containing particle(s) according to the invention, such particles can be formed using any way known or to be developed in the art. Two exemplary preferred methods of accomplishing this include wherein (i) the non-solvent residing in the vessel in the method embodiment described above is cooled to close to its freezing point or to its freezing point prior to the addition of the polymer solution such that the polymer droplets freeze upon contact with the pre-cooled non-solvent; or (ii) the polymer droplets are frozen by contacting them with a liquefied gas such as nitrogen, which is placed over a bed of pre-frozen non-solvent (see, Fig. 2). In method (ii), after the nitrogen evaporates, the non-solvent slowly thaws and the microspheres in their frozen state will sink into the liquid, cold non-solvent where the extraction process (removal of the polymer solvent) will be carried out.

By modifying this general process, one may prepare particles that are hollow or substantially hollow or porous. For example, if the removal of the solvent from the bead is carried out quickly, e.g., by applying a vacuum during the final stage of incubation, porous beads will result.

The particles of the invention can be prepared in any size desired, "Microspheres" may be obtained by nebulizing the polymer solution into a polymer aerosol using either pneumatic or ultrasonic nozzles, such as, for example a Sonotek 8700-60ms or a Lechler US50 ultrasonic nozzle, each available from Sono[.tek] Corporation, Milton, New York, U.S.A. and Lechler GmbH, Metzingen, Germany. Larger particles may be obtained by dispensing the droplets into the non-solvent solution using a syringe or other drop-forming device. Moreover, as will be known to a person of skill in the art, the size of the particle may also be altered or modified by an increase or decrease of the initial concentration of the polymer in the polymer solution, as a higher concentration will lead to an increased sphere diameter.

The particles described herein include a standard and/or a preferred core based on an acrylic polymer or copolymer with a shell of PTFEP. Such particles can provide a preferred spherical shape and improved specific gravity for use in a suspension of contrast media for embolization. The acrylic polymer based polymers with PTFEP shell described herein provide a substantially spherical shape, mechanical flexibility and compressibility, improved specific gravity properties. The core polymers may be formed using any acceptable technique known in the art, such as that described in B. Thanoo et al., "Preparation of Hydrogel Beads from Crosslinked Poly(Methyl Methacrylate) Microspheres by Alkaline Hydrolysis," J. Appl. P. Sci., Vol. 38, 1153-1161 (1990). Such acrylic-based polymers are preferably formed by polymerizing unhydrolyzed precursors, including methyl acrylate (MA), methyl methacrylate (MMA), ethylmethacrylate (EMA), hexamethyl (HMMA) or hydroxyethyl methacrylate (HEMA), and derivatives, variants or copolymers of such acrylic acid derivatives. Most preferred is MMA. The polymer is present in the core in a hydrated or partially hydrated (hydrogel) form. Such polymers are preferably cross- linked in order to provide suitable hydrogel properties and structure, such as enhanced non- biodegradability, and to help retain the mechanical stability of the polymer structure by resisting dissolution by water.

Preferably, the core prepolymers are formed by dispersion polymerization that may be of the suspension or emulsion polymerization type. Emulsion polymerization results in substantially spherical core particles of about 10 nm to about 10 microns. Suspension polymerization results in similar particles but of larger sizes of about 50 to about 1200 microns.

Suspension polymerization may be initiated with a thermal initiator, which may be solubilized in the aqueous or, more preferably, monomer phase. Suitable initiators for use in the monomer phase composition include benzoyl peroxide, lauroyl peroxide or other similar peroxide-based initiators known or to be developed in the art, with the most preferred initiator being lauroyl peroxide. The initiator is preferably present in an amount of about 0.1 to about 5 percent by weight based on the weight of the monomer, more preferably about 0.3 to about 1 percent by weight based on the weight of the monomer. As noted above, a cross-linking co- monomer is preferred for use in forming the hydrated polymer. Suitable cross-linking co- monomers for use with the acrylic-based principle monomer(s) used in preparing a polymerized particle core, include various glycol-based materials such as ethylene glycol dimethacrylate (EGDMA), diethylene glycol dimethacrylate (DEGDMA) or most preferably, triethylene glycol dimethacrylate (TEGMDA). A chain transfer agent may also be provided if desired. Any suitable MA polymerization chain transfer agent may be used. In the preferred embodiment herein, dodecylmercaptane may be used as a chain transfer agent in amounts acceptable for the particular polymerization reaction.

The aqueous phase composition preferably includes a surfactant/dispersant as well as a complexing agent, and an optional buffer is necessary. Surfactants/dispersants should be compatible with the monomers used herein, including Cyanamer^{®} 370M, polyacrylic acid and partially hydrolyzed polyvinyl alcohol surfactants such as 4/88, 26/88, 40/88, A dispersant should be present in an amount of about 0.1 to about 5 percent by weight based on the amount of water in the dispersion, more preferably about 0.2 to about 1 percent by weight based on the amount of water in the dispersion. An optional buffer solution may be used if needed to maintain adequate pH. A preferred buffer solution includes sodium phosphates (Na₂HPO₄/NaH₂PO₄). A suitable complexing agent is ethylene diamine tetraacetic acid (EDTA), which may be added to the aqueous phase in a concentration of from about 10 to about 40 ppm EDTA, and more preferably about 20 to about 30 ppm. It is preferred that in the aqueous phase composition, the monomer to water ratio is about 1:4 to about 1:6.

The polymerization should take place at about ambient conditions, preferably from about 60° C to about 80° C with a time to gelation of about one to two hours. Stirring at rates of 100 to 500 rpm is preferred for core particle formation, with lower rates applying to larger sized core particles and higher rates applying to smaller sized core particles.

Once PMMA core particles, such as microparticles, are formed, they are preferably subjected to hydrolysis conditions typical of those in the art, including use of about 1-10 molar excess of potassium hydroxide per mol of PMMA. Such potassium hydroxide is provided in a concentration of about 1-15% potassium hydroxide in ethylene glycol. The solution is then heated preferably at temperatures of about 150-185° C for several hours. Alternatively, to minimize reactant amounts and cost, it is preferred that lesser amounts of potassium hydroxide be used which are less than about 5 molar excess of potassium hydroxide per mole of PMMA, more preferably about 3 molar excess or less. For such hydrolytic reactions, a concentration of about 10-15% potassium hydroxide in ethylene glycol is also preferably used, and more preferably about 14% to about 15%. It will be understood by one skilled in the art, that heating conditions at higher temperatures may be used to decrease overall reaction times. Reaction times may be varied depending on the overall diameter of the resultant core particles. For example, the following conditions are able to provide particles having about 35% compressibility and desired stability: for diameters of about 200-300 µm, the solution should be heated for about 7.5 to about 8.5 hours; for diameters of about 300-355 µm, about 9.5 to about 10.5 hours; for diameters of about 355-400 µm, about 11.5 to about 12.5 hours; and for about 400-455 µm, about 13.5 to about 14.5 hours, etc. The core particle size can be adjusted using variations in the polymerization process, for example, by varying the stirring speed and the ratio of the monomer to the aqueous phase. Further, smaller sizes can be achieved by increasing surfactant/dispersant ratio.

Following hydrolysis, core particles are separated from the reaction mixture and their pH may be adjusted to any range as suited for further processing steps or intended uses. The pH of the particle core may be adjusted in from about 1.0 to about 9.4, preferably about 7.4 if intended for a physiological application. Since size, swelling ratio and elasticity of the hydrogel core material are dependent on pH value, the lower pH values may be used to have beneficial effects during drying to prevent particle agglomeration and/or structural damage. Core particles are preferably sieved into different size fractions according to intended use. Drying of particles preferably occurs using any standard drying process, including use of an oven at a temperature of about 40° -80° C for several hours up to about a day.

To provide desired surface properties to the hydrophilic hydrogel core particles, in order to provide adhesion for receiving a PTFEP coating, the surface of the hydrogel may be subjected to treatment with any suitable ionic or non-ionic surfactant, such as tetraalkylammonium salts, polyalcohois and similar materials. A more permanent change in adhesion properties is brought about by rendering the surface of the core particles hydrophobic by reaction of its polymethacrylic acid groups with a suitable reactant. Suitable reactants include, but are not limited to, hydrophobic alcohols, amides and carboxylic acid derivatives, more preferably they include halogenated alcohols such as trifluoroethanol. Such surface treatment also prevents delamination of the coating from the core once the coating is applied. Preferred surface treatments may include, without limitation, an initial treatment with thionyl chloride followed by reaction with trifluoroethanol. Alternatively, the surface may be treated by suspending the core particles in a mixture of sulfuric acid and a hydrophobic alcohol, such as trifluoroethanol. Such treatments are preferred if the core particles are to be coated in that they minimize any delamination of a coating.

Alternatively, and most preferably, the PMA core particles may be coated with a surface layer of and/or infused with barium sulfate. The barium sulfate is radiopaque and aids in visualization of the finished particles when in use. It also provides enhanced fluidization properties to the particles such that it reduces agglomeration especially during drying and allows for fluid bed coating of the PMA core particles with an outer coating of PTFEP, thereby providing improved adhesion between a PTFEP outer core and a polymeric acrylate core particles. By allowing fluidization even when the core particles are swollen, barium sulfate also improves the overall coating and adhesion properties. By enabling the coating of the core particles even in a swollen state with PTFEP, barium sulfate also reduces the potential tendency of the PTFEP shells to crack or rupture in comparison with coating the core particles in a dry state and then later exposing the particles to a suspension in which the core particles swell and exert force on the shell of PTFEP. A coating of barium sulfate on the core particles is preferably applied by adhesion of the barium sulfate in the form of an opaque coating on the hydrogel surface of the PMA beads. Barium sulfate can further assist in reducing electrostatic effects that limit particle size. By allowing for absorption of additional humidity, the barium sulfate tends to counteract the electrostatic effects.

Barium sulfate crystals adhering only loosely to the PMA core particles may be covalently crosslinked or chemically grafted to the core particle surface by spraycoating a sufficient amount of an aminosilane adhesion promoter onto the PMA core particle. This will help to effectively reduce barium sulfate particulate matter in solution after hydration of the core particles. Exemplary core particles include 3-ammopropyl-trimethoxysilane and similar silane-based adhesion promoters.

For improving visualization of microparticles made as noted herein include the absorption of a water soluble organic dye inside the hydrogel core particles. Exemplary dyes are preferably those FDA dyes approved for human use and which are known or to be developed for safe, non-toxic use in the body and which are capable of providing acceptable contrast. Organic dyes may include dyes such as D&C Violet no. 2 and others preferably approved for medical device uses, such as for contact lenses and resorbable sutures. Whereas barium sulfate operates as an inorganic filler and finely dispersed pigment that makes the particles visible by light diffraction due to small crystal size, the dyes when impregnated in the particles absorb the complementary part of the visible color spectrum.

The microparticles made in accordance with the foregoing process for forming a core hydrogel polymer are then coated with PTFEP and/or its derivatives. Any suitable coating process may be used, including solvent fluidized bed and/or spraying techniques. However, preferred results may be achieved using fluidized bed techniques in which the core particles pass through an air stream and are coated through spraying while they spin within the air stream. The PTFEP or derivative polymer is provided in dilute solution for spraying to avoid clogging of the nozzle.

Exemplary solvents for use in such solutions include ethyl acetate, acetone, hexafluorbenzene, methyl ethyl ketone and similar solvents and mixtures and combinations thereof, most preferred is ethyl acetate alone or in combination with isoamyl acetate. Typical preferred concentrations include about 0.01 to about 0.3 weight percent PTFEP or its derivative in solution, more preferably about 0.02 to 0.2 weight percent PTFEP, and most preferably about 0.075 to about 0.2 weight percent. It should be understood based on this disclosure that the type of hydrogel core can be varied as can the technique for coating a core particle, however it is preferred that a core which is useful in the treatment techniques and applications described herein is formed and subsequently coated with PTFEP and/or its derivatives as described herein.

As previously discussed, the particles can be used in various medical and therapeutic applications, such as embolization, drug delivery, imaging (ultrasound) and as tracer particles. For example, in one embodiment, the invention includes particles for use in a method of minimizing blood flow to a specific tissue in a mammal. This process, commonly referred to as embolization, includes occluding or obstructing at least a portion of a vessel, or the entire vessel, with one or more of the particles of the invention. Such procedure is particularly useful in the treatment of diseases and pathologies that involve undesirable vascularized tissues, for example, tumor tissue or disorders involving the uncontrolled proliferation of certain ceils such as endometriosis. In such procedures, the particle(s) are prepared in accordance with the procedures described above, and may be inserted into the blood vessel by any invasive or non- invasive medical practice known or to be developed in the art such as via a catheter, a syringe, or a surgical incision. The embolization can be carried out such that only a portion of the blood vessel is occluded, or the entire vessel may be occluded. In the method, if desired, one may use particles that have been loaded with an active agent such as a cytostatic agent, an anti-inflammatory agent, an anti-mitogenic or cell proliferation active agent, a hormone, or any other desirable active agent, as described herein. Embolization particles according to the present invention are capable of demonstrating improved optical visibility, additional radiopacity, and an optimum specific density of about 1.17 g/cm³. The embolization particles in this invention may be used with different dyes as markers as noted above for particle sizes, embedded pharmaceuticals for localized drug delivery and controlled drug elution characteristics.

For use in embolization therapy, particle density is preferably taken into consideration to ensure beneficial properties for particle delivery. Possible clogging of a catheter-based delivery system may occur if using a density-mismatched delivery medium. In addition, it is desirable to include a certain minimum amount of contrast agent in the delivery medium to achieve sufficient levels of fluoroscopic contrast during surgery. Currently, the polymethacrylate hydrogel density is between 1.05 g/c m³ and 1.10 g/c m³ depending on the equilibrium water content. The most common iodinated nonionic contrast agent media with 300 mg iodine per ml have densities of 1.32-1.34 g/c m³. As used herein, "buoyancy" refers to the ability of the particles to be substantially free floating in solution that occurs when the density of the particle is substantially the same as the medium in which it is suspended. Coated particles formed in accordance with the present invention as described herein can reach buoyancy when there is approximately 30% contrast agent in the delivery medium, however, such levels can be adjusted for such preferred use according to techniques described herein.

One method for increasing the density of the particles is by use of heavy water or deuterium oxide (D₂O). When heavy water is used to swell the particles, D₂O displaces H₂O, thereby increasing the weight of the particles for better dispersion and buoyancy levels. Typically this leads to the ability to add higher amounts of contrast agent of at least about 5% using such a technique. However, some equilibrating effect can occur over time when the particles are contacted with an aqueous solution of contrasting agent. Thus, it is preferred that when using D₂O for this purpose, either that suspension times are kept to a minimum or, more preferably, that the contrast agent be provided in a solution which also uses D₂O.

Alternatively, particles of pH 1 can be neutralized with cesium hydroxide and/or the final neutralized particles can be equilibrated with cesium chloride. Such compounds diffuse cesium into the particles, such that either the cesium salt of polymethacrylic acid is formed or polymethacrylic acid is diffused and thereby enriched with cesium chloride.

The cesium increases the density of the particles, thereby increasing the ability to add higher amounts of contrast agent. Typical buoyancy levels can be adjusted using the cesium technique such that about 45 to about 50% contrast agent may be added to the delivery medium as is desired for embolization. Cesium salts are non-toxic and render the particles visible using fluoroscopy. Cesium's atomic weight of 132.9 g/mol is slightly higher than that of iodine providing beneficial effects including increase in overall density and enhancement of X-ray contrast visibility even without a contrast agent. For certain cancer treatments where a radioactive isotope of cesium is desired, such active agent can be used as an alternative cesium source rendering the particles buoyant in an embolic solution as well as able to be used as an active treatment source.

The above-noted techniques for improving density of particles, such as microparticles for embolization or other applications where density and/or buoyancy in solution are applicable properties may be applied in to the preferred particles described herein and/or may be applied for other similar particles. It should be understood that the disclosure is not limited to cesium and/or D2O treatment of the preferred particles herein and that such techniques may have broader implications in other particles such as other acrylic-based hydrogels and other polymeric particles.

As noted above, barium sulfate may be used between the core particles and the preferred PTFEP coating or introduced into the interior of the core particles using any technique known or to be developed in the art. Also, organic dyes may similarly be included in the particle core. These materials, particularly the barium sulfate, also contribute to an increase in density as well as providing radiopacity. In addition to a general density increase as provided by the above-noted D2O or cesium compounds, the barium sulfate allows this benefit even upon substantial and/or full hydration, allowing particles in suspension to remain isotonic. Thus, a barium sulfate powder coating can provide an inert precipitate having no effect on physiological osmolarity.

It should be understood, based on this disclosure, that the various buoyancy additives noted above can be used independently or in combination to provide the most beneficial effects for a given core particle and coating combination.

The invention also includes particles for use in methods of delivering an active agent to a localized area within the body of a mammal. The method includes contacting the localized area with at least one of the particles of the invention as described above, such that an effective amount of the active agent is released locally to the area. Diseases or pathologies that may be treated by this method include any wherein the localized or topical application of the active agent achieves some benefit in contrast to the systemic absorption of the drug. Suitable active agents include NSAIDS, steroids, hormones, nucleic acids, agents used in the treatment of disorders of the gastrointestinal tract, such as, ulcers, Crohn's disease, ulcerative colitis, and irritable bowel syndrome. Other active agents may include tacrolimus, sirolimus, paclitaxel, cis-/carboplatins, antineoplastic agents, doxorubicine and/or receptor blocking agents, e.g., avβ3 integrin blockers, which inhibit cell attachment.

If the particle formulated for delivery of an active agent to a localized area is about 1 to about 1,000 µm in diameter, the drug loaded microspheres can be applied to localized areas within the mammalian body using syringes and/or catheters as a delivery device, without causing inadvertent occlusions. For example, using a contrast agent, a catheter can be inserted into the groin artery and its movement monitored until it has reached the area where the localized administration is desired. A dispersion of the particles in a suitable injection medium can be injected through the catheter, guaranteeing only a specific area of the body will be subjected to treatment with drug loaded beads (particles), As will be understood to a person of skill in the art, injection mediums include any pharmaceutically acceptable mediums that are known or to be developed in the art, such as, *e.g.,* saline, PBS or any other suitable physiological medium. In accordance with a further embodiment described herein, the invention includes an injectable dispersion including particles and a contrasting agent which particles are substantially dispersed in the solution. In a preferred embodiment, the particles are also detectible through fluoroscopy.

The polymeric particles of the invention may be used to prepare a sustained release formulation of an active agent for oral administration. The formulation comprises a particle, as described above, loaded with an active agent. The polymeric particle utilized may be hollow, substantially hollow or solid. The particle can be loaded with the active agent either by dispersion or solvation of the active agent in the polymer solution prior to the production of micro-sized particles through spray droplets, pastillation of a polymer melt or carrying out of a cryoextraction process. Alternatively, an unloaded polymer particle can be prepared and subsequently immersed in solutions containing active agents. The particles are then incubated in these solutions for a sufficient amount of time for the active agent to diffuse into the matrix of the polymer. After drying the particles, the active agent will be retained in the polymer particle. If this loading mechanism is utilized, drug loading can be controlled by adjusting drug concentrations of the incubation medium and removing the particles from the incubation medium when an equilibrium condition has been attained.

Moreover, it is envisioned that the active agent can be selected so as to complement the action of the particles in a synergistic fashion, especially if the particles are being used in an occlusive or embolization procedure. For example, if the tissue to which one wishes to minimize blood flow is a tumor tissue, one may wish to load the particles used in the occlusion with a cytostatic drug, antiangiogenic agents, or an antimitotic drug.

Also provided is a method of tracing the passage of a particle through a blood vessel or other cavity in a mammalian body. The method includes injecting into the vessel, cavity, or a conduit adjacent to such cavity or vessel, at least one tracer particle, wherein the tracer particle is at least a particle prepared in accordance with the procedures described above.

The tracer particle may include a contrast agent that may aid in the visualization of the particle as it passes through the body cavity, blood vessel, and/or other locale. In general, in this application smaller particles are preferred, such as those in the range of about 1 to about 10 µm, especially if the particles are to be injected into the bloodstream. However, the particles may be of any size so long as, for this purpose, they are not large enough to occlude the blood vessel, body cavity, or adjacent cavity or vessel to which the procedure is being applied.

If the particles are loaded with a contrast agent, their movement can be visualized with X-ray machines, or any other contrasting procedure, depending on the contrast agent utilized. However, if the particles do not contain a contrast agent, the flow of the particles may be visualized using ¹⁹F-NMR based computer tomography.

If desired, one may coat the tracer particle containing a contrast agent with a polymer coating. The polymer coating may comprise any polymer known or to be developed in the art, including any phosphazene polymers. If there is any toxicity or concern of toxicity with respect to the contrast agent, it is desirable that the one or more coating is nonbiodegradable. Depending on the nature of the visualization procedure, such contrast agents may be provided (e.g., from the class of conventional radiographic contrast enhancing agents such as ionic or nonionic Iodine-containing compounds (Imeron^{™}, Optiray^{™}, etc.).

Where magnetic resonance imaging (MRI) is employed for visualization, the contrast agent to be provided may be chosen from the class of rare earth compounds, such as Gadolinium and Samarium-chelates, and so forth, as is well known to the art.

Since the hydrogel core component in embodiments of the present invention is derived from an anionic acrylate-based hydrogel polymer, such as Polymethacrylic acid and the like, the incorporation of multivalent metal compounds, including aforementioned rare earth or other metals, facilitates a favorable ionic interaction of these compounds, such as by ionic crosslinking or similar ionic interaction, thus providing for favorable retention or accumulation of these compounds in the particles and hence providing for a sustained release effect of such compounds in various embodiments according to the present invention.

The invention also includes particles for use in the method of carrying out an enhanced ultrasound imaging procedure (sonography). In order to do this, one may administer to the ultrasound subject at least one hollow microcapsule to the area of the ultrasound subject that one wishes to visualize. Such administration can be accomplished by any means known or to be developed in the art, including by use of a syringe, catheter or other invasive or non-invasive medical device, and/or by a surgical incision. In such method, it is preferable to use particles which are hollow or substantially hollow, i.e. having an inner cavity that is equal to at least about 20%, at least about 30%, at least about 40%, at least about 50%, at least about 80%, at least about 90%, of the volume of the entire particle. The hollow particles are administered to a portion of the ultrasound subject which one wishes to image. While not wishing to be bound by theory, it is speculated that the particles enhance the ultrasound image by increasing the ultrasound "echo" due to their abrupt density change, when compared to the surrounding tissue. The hollow cavities of the particles act to reflect the ultrasound, thereby enhancing the image.

Unless indicated otherwise, temperature is reported in degrees Centigrade and pressure is at or near atmospheric. An example of the preparation of a polyphosphazene of this invention is provided with the synthesis of poly[bis(trifluoroethoxy)phosphazene] (PzF) polymer, which may be prepared according to U.S. Patent Application Publication No. 2003/0157142.

Also unless indicated otherwise, when a range of any type is disclosed or claimed, for example a range of molecular weights, layer thicknesses, concentrations, temperatures, and the like, it is intended to disclose or claim individually each possible number that such a range could reasonably encompass, including any sub-ranges encompassed therein. For example, when the Applicants disclose or claim a chemical moiety having a certain number of atoms, for example carbon atoms, Applicants' intent is to disclose or claim individually every possible number that such a range could encompass, consistent with the disclosure herein. Thus, by the disclosure that an alkyl substituent or group can have from 1 to 20 carbon atoms, Applicants intent is to recite that the alkyl group have 1, 2, 3, 4, 5, 6, 7, 8, 9, 10, 11, 12, 13, 14, 15, 16, 17, 18, 19, or 20 carbon atoms. In another example, by the disclosure that microspheres have a diameter of approximately 500 to 600 µm, Applicants include within this disclosure the recitation that the microspheres have a diameter of approximately 500 µm, approximately 510 µm, approximately 520 µm, approximately 530 µm, approximately 540 µm, approximately 550 µm, approximately 560 µm, approximately 570 µm, approximately 580 µm, approximately 590 µm, and/or approximately 600 µm, including any range or sub-range encompassed therein.

### EXAMPLE 1 (Reference)

Microparticle cores were formed in accordance with a preferred embodiment herein. A deionized water solution of polyvinyl alcohol (PVA) was prepared using about 23g of PVA of weight average molecular weight of about 85,000-124,000, which PVA was about 87-89% hydrolyzed and 1000 g water. A phosphate buffer solution was prepared using 900 g deionized water, 4.53 g disodium hydrogen phosphate, 0.26 g sodium dihydrogen phosphate and 0.056 g ethylenediamine tetraacetic acid (EDTA). Methyl methacrylate (MMA) monomer was vacuum distilled prior to use.

Polymerization was carried out in a three-necked, round- bottomed, 2000-mi flask with a KPG mechanical stirring apparatus attached. The flask was also equipped with a thermometer, reflux condenser and a pressure release valve with a nitrogen inlet. The polymerization process further utilized 100 ml of the PVA solution prepared above, 900 ml of the phosphate buffer solution, 0.65 g of dilauroyl peroxide, 200.2 g methacrylic acid methyl ester and 2.86 g triethylene glycol dimethacrylate. [00136] The PVA and buffer solutions were provided to the reactor flask. The distilled MMA and triethylene glycol dimethacrylate were introduced, dilauroyl peroxide then added to the same flask and the components were agitated to ensure dissolved solids. The reaction flask was flushed with argon and the stirrer speed set to at 150 rpm to produce particle sizes of a majority in the range of 300-355 µm. Stirring continued for approximate 5 minutes. The stirrer was then set to 100 rpm and argon flushing was discontinued. The reaction flask was then subjected to a water bath which was heated to 70° C and held at approximately that temperature for about 2 hours. The temperature of the bath was then increased to 73° C and held for an hour, then the water bath temperature was raised again to 85° C and held for another hour. The stirring and heat were discontinued. The solution was filtered and the resulting polymethylacrylate microparticle cores were dried in an oven at 70° C for about 12 hours. The microparticle cores were subjected to sieving and collected in size fractions of from 100-150; 150-200; 200-250; 250-300; 300-355; 355-400; and 400-450 µm with a maximum yield at 300-355 µm.

The PMMA microparticle cores thus formed were then hydrolyzed. A portion of 100 g 250-300 µm sized microparticle cores, 150 g potassium hydroxide and 1400 g of ethylene glycol were added to a 2000 ml flask, reflux condenser with drying tube connected, and the mixture was heated at 165° C for 8 hours for full hydrolysis. The mixture was allowed to cool to room temperature, solution decanted and the microparticle cores were washed with deionized water. The procedure was repeated for other calibrated sizes of microparticles (the following reaction times applied: 300-355 micron particles: 10 hours; 355-400 micron particles: 12 hours and 400-455 micron particles: 14 hours). That is, the particular size of the particles can be selected, standardized, or calibrated according to the conditions under which they are prepared.

The microparticle cores were finally acidified with hydrochloric acid to a pH of 7.4, and dried in an oven at approximately 70° C.

### EXAMPLE 2 (Reference)

Microparticle cores formed in accordance with Example 1 were then esterified in this Example. For esterification surface treatment, 800 g of dried microparticle cores from Example 1 were weighed in a 2L reaction vessel with a reflux condenser. 250 g thionyl chloride in 1.5 L diethyl ether were added under stirring. Stirring was continued at room temperature for 20 hours. The solvent and volatile reactants were removed by filtration and subsequent vacuum drying. Then 500 g trifluoroethanol in 1.5 L ether were introduced and the suspension stirred for another 20 hours at room temperature. The particle cores were finally dried under vacuum.

### EXAMPLE 3 (Reference)

In an alternative surface treatment to Example 2, 800 g dried microparticle cores from Example 1 were reacted with 1140 g trifluoroethanol and 44 g sulfuric acid added as a catalyst. The mixture was stirred for 20 hours at room temperature, filtered and dried under vacuum.

### EXAMPLE 4 (Reference)

800 g of dry PMMA potassium salt microparticle cores which were partially esterified with trifluoroethanol as described above in Examples 1-2 were spray coated with PTFEP in an MP-I Precision Coater™ fluidized bed coating apparatus (available from Aeromatic- Fielder AG, Bubendor, Switzerland). The particle cores were picked up by an air stream (40-60 m³/h, 55° C incoming temperature) and spray coated with PTFEP solution microdroplets from an air-fluid coaxial nozzle. The solution composition was 0.835 g PTFEP, 550 g ethyl acetate and 450 g isopentyl acetate. It was fed through the nozzle's 1.3 mm wide inner bore at a rate of 10-30 g/min. At the nozzle head, it was atomized with pressurized air (2.5 bar). The total amount of spray solution (3kg) was calculated to coat the particle core with a 150 nm thick PTFEP film.

### EXAMPLE 5 (Reference)

The absorption of organic dyes was tested on microparticles formed according to Example 1. To 2 ml of phosphate buffered saline solution containing 1 ml of hydrated beads was provided an amount of 5-10 µl of the respective dye as a 10 millimolar solution in ethanol. The samples were incubated for 30-60 minutes at room temperature under gentle shaking of the vial. Supernatant liquid was discarded and particles were washed three times with 2 ml of either deionized water, saline or PBS buffer solution prior to visualization with optical and fluorescence microscopy. The dyes tested included triphenylmethane derived dyes such as Fluoescein diacetate and Rhodamin 6G which were evaluated along with carbocyanine based dyes such as Dil The triphenylmethane based Fluorecein and Rhoamine dyes exhibited a specific affinity for the hydrophilic PMMA hydrogel core through ionic interactions. They were able to easily withstand the rigorous conditions of repeated washing and steam sterilization without substantial leaching. The carbocyanine dye Dil on the other hand exhibited a high selectivity for the hydrophobic PTFEP shell, without penetrating the hydrophilic PMAA core material. Thus with the subsequent staining employing the combination of Dil and Fluorescein diacetate both core and shell could be simultaneously visualized employing a fluorescence optical microscope. As a result, this procedure provides a fast, sensitive fluorescence-staining assay for the PMAA particles that makes core and shell simultaneously visible under conditions encountered in actual application.. This procedure further enables assessment of the mechanical-elastic stress or damage to the PTFEP shell. It further shows the affinity of certain classes of dyes for the various components of the particle.

Use of these and other dyes may be used to visually identify selected microspheres, which may be provided and dyed for identification to indicate certain sizes of microspheres for use in selected clinical or diagnostic applications. Color-coding may also be used to identify selected microspheres on the basis of other properties, such as content of certain therapeutic or diagnostic agents. Applications according to the present invention may also improve the imaging visualization by enhancing the particles' buoyancy behavior

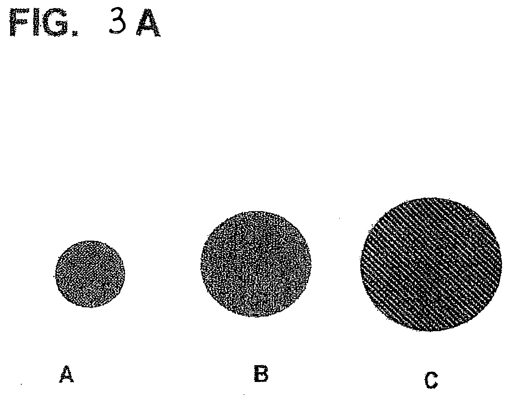

Fig. 3A shows exemplary microspheres A, B, and C of the present invention, in which the microspheres are each of different diameters, and each has a different color-coding. In an exemplary use of such microspheres of the present invention, color-coded microspheres of like sizes may be separately packaged and supplied for use. Such color-coded microspheres may provide a user a visual indication of the specific microsphere in a particular clinical or diagnostic use.

In various embodiments according to the present invention, microspheres may be produced in calibrated sizes ranging from about 1 to about 10,000 nanometers in diameter. In one embodiment of the present invention, microspheres of the present invention may be provided in sizes of about 40, about 100, about 250, about 400, about 500, about 700, and about 900 nanometers in diameter, with a visually distinctive color imparted to each size of microsphere. Other sizes, size ranges, and calibrated sized microspheres lacking color dye are also included in the present invention. Not only may the microspheres or particles be provided in different size ranges, but their elasticity may be controlled according to the present invention to specifically provide for proximal or distal embolization behavior, due to potentially differing ranges of compressibility which may alter the traveling distance of the particles or microspheres upon their release within a selected blood vessel. Microspheres of the present invention may also be provided in customized sizes and/or with customized colors as specified by a user for specific clinical diagnostic or therapeutic applications.

### EXAMPLE 6 (Reference)

Transarterial chemoembolization or TACE is a clinical procedure in which the blood supply to a tumor is disrupted by embolization and chemotherapy is administered directly into the tumor. Selective embolization of tumor blood vessels without direct administration of chemotherapy (bland embolization) is also preformed as a clinical procedure in certain situations.

In most living organisms with a developed circulatory system, the vasculature tends to taper from larger diameter vessels proximal to the heart to smaller vessels more distal to the heart. Larger arteries thus tend to divide into smaller arteries, which eventually taper to the arteriole level and interface with small diameter venules. Venous flow progresses from such venules through successively larger diameter veins as flow returns to the heart.

It is common, therefore, that blood vessels of differing sizes may exist within a tumor mass or other target tissue. In a clinical situation where embolization and maximal disruption of blood supply to a tumor or other target tissue is desired, serial embolization of progressively larger tumor vessels may provide a more complete embolization, with or without the delivery of chemotherapeutic or other therapeutic agents.

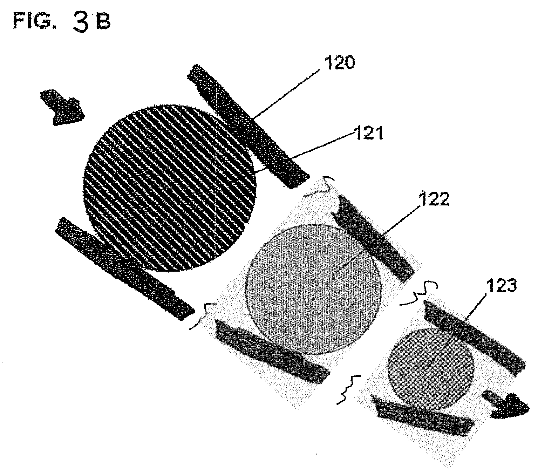

Fig. 3B is a conceptual representation of a selective embolization of an exemplary artery 120 by serial administration of different sized microspheres 121, 122, and 123. The direction of blood flow within the exemplary artery 120 is shown by the arrows in Fig. 3B. In this example, microsphere 121 is the smallest diameter of the microspheres administered, and is injected into artery 120 first, occluding the vessel lumen at the smallest vessel diameter that will not permit passage of microsphere 121. Continuing in this example, microsphere 122 is of intermediate diameter of the microspheres administered, and is injected into artery 120 first, occluding the vessel lumen at the smallest vessel diameter that will not permit passage of microsphere 122. Finally, in this example, microsphere 123 is the largest diameter of the microspheres administered, and is injected into artery 120 first, occluding the vessel lumen at the smallest vessel diameter that will not permit passage of microsphere 123. The result in this example is the sequential blockage of blood flow at multiple levels throughout the blood supply of the tumor or target tissue.

In other examples of the present invention, less than three or more than three different sized microspheres may be administered to secure the desired embolization of a tumor or other target tissue.

As provided in previous examples of the present invention, different-sized microspheres of the present invention may further be provided with color-coding to allow user identification and visual confirmation of the sized microspheres in use at any given stage of the clinical procedure.

The delivery of microspheres of different sizes or other inherent qualities may further be facilitated by the use of transport packaging and/or delivery devices which are color- coded to allow user identification and visual confirmation of the sized microspheres in use at any given stage of the clinical procedure in exemplary applications according to the present invention. In various exemplary applications of the present invention, such color-coded devices may be used in combination with color-coding of the microspheres themselves, with corresponding microsphere and packaging/delivery device color-coding.

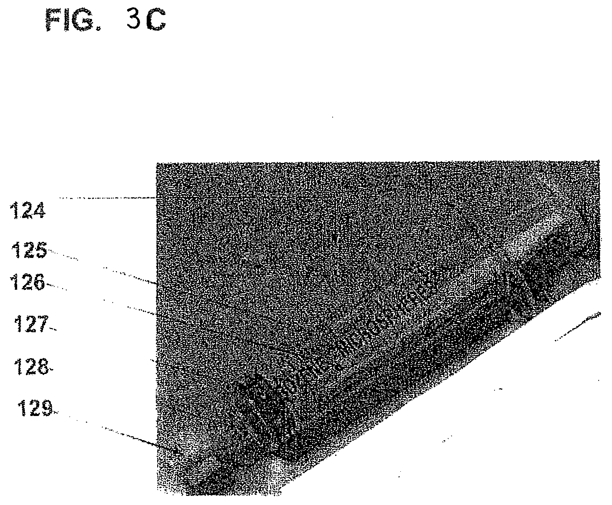

Fig. 3C shows a syringe used for the packaging and/or delivery of color-coded microspheres of a select size according to the present invention. In the example shown in Fig. 3C, the syringe 124 comprises a barrel 125, a plunger 126, a plunger tip 127, a Luhr-type injection tip 128, and a Luhr tip cover 129.

As shown in Fig. 3C, one or more of components barrel 125, a plunger 126, a plunger tip 127, a Luhr-type injection tip 128, and a Luhr tip cover 129 may be colored in a common color according to a color code to indicate a desired property of the microspheres contained therein. In one example of the present invention, a syringe may contain color-coded microspheres to indicate a certain microsphere size, and the syringe plunger, plunger tip, and Luhr tip cover may be similarly colored to further indicate the desired property of the contained microspheres to a user.

## Claims

1. Color-coded polymeric particles comprising:
an anionic acrylate-based hydrogel core;
a multivalent metal compound that forms an ionic interaction with the acrylate-based hydrogel core; and
an outer shell comprising one or more dyes and a polyphosphazene having the general formula (I): 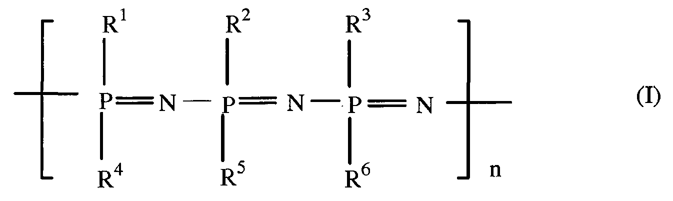
in which the n value is an integer from 2 to ∞;
R¹ to R⁶ are independently selected from the group consisting of:
a substituted or unsubstituted alkyl, aminoalkyl, haloalkyl, thioalkyl, thioaryl, alkoxy, haloalkoxy, aryloxy, haloaryloxy, alkylthiolate, arylthiolate, alkylsulphonyl, alkylamino, dialkylamino, heterocycloalkyl comprising one or more heteroatoms selected from nitrogen, oxygen, sulfur, phosphorus, or a combination thereof, or heteroaryl comprising one or more heteroatoms selected from nitrogen, oxygen, sulfur, phosphorus, and a combination thereof
wherein the polymeric particles are formed as microspheres, each microsphere being produced in a size or size range and each size or size range having a visually distinctive color imparted to it by the one or more dyes to provide a visual indication of the size or size range of the specific microsphere.

2. The color-coded polymeric particles of Claim 1, wherein R¹ to R⁶ are selected independently from the group consisting of OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1% or less OCH₂CH=CH₂, 1% or less OCH₂CH₂CH=CH₂, and any combinations thereof.

3. The color-coded polymeric particles of Claim 1, wherein the polyphosphazene is poly[bis(2,2,2-trifluoroethoxy)]phosphazene or a derivative of poly[bis(2,2,2-trifluoroethoxy)]phosphazene.

4. The color-coded polymeric particles of Claim 1, wherein the acrylate-based hydrogel core comprises a polymer selected from the group consisting of poly(methacrylic acid), poly(acrylic acid), copolymers thereof, and combinations thereof.

5. The color-coded polymeric particles of Claim 1 calibrated to have an average diameter selected from the group consisting of: from 1 µm to 5,000 µm from 40 µm to 5,000 µm, from 1 to 1,000 µm, from 200 to 500 µm, from 1 to 200 µm, from 40 to 1000 µm.

6. The color-coded polymeric particles of Claim 1 calibrated to have an average diameter selected from the group consisting of 40 µm, 100 µm, 250µm, 400 µm, 500 µm, 700 µm, 900 µm and 1000 µm.

7. The color-coded polymeric particles of claim 1 are bioabsorbable or nonbioabsorbable.

8. The color-coded polymeric particles of claim 1, wherein the core further comprises one or more active agents.

9. The color-coded polymeric particles of Claim 1, wherein the acrylate-based hydrogel core comprises an agent of interest selected from the group consisting of barium sulfate, a pharmaceutical agent, a contrast agent, a steroid, a hormone, a nucleic acid, an antibiotic, an antiseptic, an analgesic, an anti-neoplastic, an anesthetic, and combinations thereof.

10. A method for making color-coded polymeric particles, the method comprising:
coating an anionic acrylate-based hydrogel core with an outer shell;
forming an ionic interaction between the acrylate-based hydrogel core and a multivalent metal compound;
wherein the outer shell comprises one or more dyes and a polyphosphazene having the general formula (I): 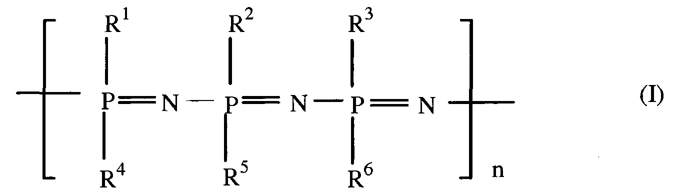
in which the n value is an integer from 2 to ∞;
R¹ to R⁶ are independently selected from the group consisting of:
a substituted or unsubstituted alkyl, aminoalkyl, haloalkyl, thioalkyl, thioaryl, alkoxy, haloalkoxy, aryloxy, haloaryloxy, alkylthiolate, arylthiolate, alkylsulphonyl, alkylamino, dialkylamino, heterocycloalkyl comprising one or more heteroatoms selected from nitrogen, oxygen, sulfur, phosphorus, or a combination thereof, or heteroaryl comprising one or more heteroatoms selected from nitrogen, oxygen, sulfur, phosphorus, and a combination thereof wherein the polymeric particles are formed as microspheres, each microsphere being produced in a size or size range and each size or size range having a visually distinctive color imparted to it by the one or more dyes to provide a visual indication of the size or size range of the specific microsphere.

11. The method of Claim 10, wherein R¹ to R⁶ are selected independently from the group consisting of OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1% or less OCH₂CH=CH₂, 1% or less OCH₂CH₂CH=CH₂, and combinations thereof.

12. The method of Claim 10, wherein the acrylate-based hydrogel core comprises a polymer selected from the group consisting of poly(methacrylic acid), poly(acrylic acid), copolymers thereof, and combinations thereof.

13. The method of Claim 10, wherein the color-coded polymeric particles are calibrated to have an average diameter selected from the group consisting of: from 1 µm to 5000 µm from 40 µm to 5,000 µm, from 1 to 1,000 µm, from 200 to 500 µm, from 1 to 200 µm, from 40 to 1000 µm.

14. The method of Claim 10, wherein the color-coded polymeric particles are calibrated to have an average diameter selected from the group consisting of 40 µm, 100 µm, 250µm, 400 µm, 500 µm, 700 µm, 900 µm and 1000 µm.

## Patentansprüche

1. Farbkodierte polymerische Teilchen, mit:
einem Acrylat-basierten Hydrogel-Kern, der ein oder mehrere Farbstoffe beinhaltet; und
einer äußeren Schale, die ein Polyphosphazen mit der allgemeinen Formel (I) umfasst: 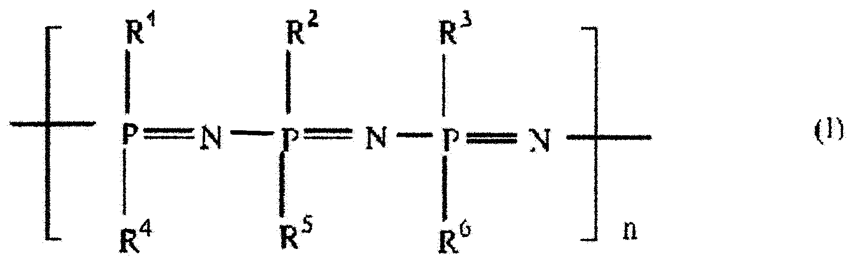 worin der n-Wert eine ganze Zahl von 2 bis ∞ ist;
R¹ bis R⁶ unabhängig aus der Gruppe ausgewählt sind, die aus:
einem substituierten oder nicht substituierten Alkyl, Aminoalkyl, Haloalkyl, Thioalkyl, Thioaryl, Alkoxy, Haloalkoxy, Aryloxy, Haloaryloxy, Alkylthiolat, Arylthiolat, Alkylsulphonyl, Alkylamino, Dialkylamino, Heterocycloalkyl mit einem oder mehreren Heteroatomen besteht, die aus Stickstoff, Sauerstoff, Schwefel, Phosphor oder einer Kombination hiervon ausgewählt sind, oder Heteroaryl, das ein oder mehrere Heteroatome umfasst, die aus Stickstoff, Sauerstoff, Schwefel, Phosphor und einer Kombination hiervon ausgewählt sind, besteht;
worin die polymerischen Teilchen als Mikrokügelchen gebildet sind, wobei jedes Mikrokügelchen mit einer kalibrierten Größe oder Größenbereich erzeugt wird, und jede Größe oder jeder Größenbereich eine optisch unterscheidbare Farbe hat, die ihm durch den einen oder mehrere Farbstoffe erteilt wird, um eine optische Anzeige der Größe oder des Größenbereiches des spezifischen Mikrokügelchen zu schaffen.

2. Die farbkodierten polymerischen Teilchen nach Anspruch 1, bei denen R¹ bis R⁶ unabhängig aus der Gruppe ausgewählt sind, die aus OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1% oder weniger OCH₂CH=CH₂, 1% oder weniger OCH₂CH₂CH=CH₂ und irgendwelchen Kombinationen hiervon besteht.

3. Die farbkodierten polymerischen Teilchen nach Anspruch 1, bei denen das Polyphosphazen Poly[bis(2,2,2-Trifluoroethoxy)]Phosphazen oder ein Derivat von Poly[bis(2,2,2-Trifluoroethoxy)]Phosphazen ist.

4. Die farbkodierten polymerischen Teilchen nach Anspruch 1, bei denen der Acrylat-basierte Hydrogel-Kern ein Polymer umfasst, das aus der Gruppe ausgewählt ist, die aus Poly(Methacryl-Säure, Poly(Acrylsäure), Copolymeren hiervon und Kombinationen hiervon ausgewählt ist.

5. Die farbkodierten polymerischen Teilchen nach Anspruch 1, die so kalibriert sind, dass sie einen mittleren Durchmesser aufweisen, der aus der Gruppe ausgewählt ist, die aus Folgendem besteht: von 1 µm bis 5,000 µm, von 40 µm bis 5,000 µm, von 1 bis 1,000 µm, von 200 bis 500 µm, von 1 bis 200 µm, von 40 bis 1000 µm.

6. Die farbkodierten polymerischen Teilchen nach Anspruch 1, die so kalibriert sind, dass sie einen mittleren Durchmesser aufweisen, der aus der Gruppe ausgewählt ist, die aus 40 µm, 100 µm, 250 µm, 400 µm, 500 µm, 700 µm, 900 µm und 1000 µm besteht.

7. Die farbkodierten polymerischen Teilchen nach Anspruch 1, die bioabsorbierbar oder nicht bioabsorbierbar sind.

8. Die farbkodierten polymerischen Teilchen nach Anspruch 1, bei denen der Kern weiterhin ein oder mehrere aktive Agenzien umfasst.

9. Die farbkodierten polymerischen Teilchen nach Anspruch 1, bei dem der Acrylat-basierte Hydrogel-Kern ein interessierendes Agens umfasst, das aus der Gruppe ausgewählt ist, die aus Bariumsulfat, einem pharmazeutischen Agens, einem Kontrast-Agens, einem Steroid, einem Hormon, einer Nukleinsäure, einem Antibiotikum, einem Antiseptikum, einem Analgesikum, einem Anti-Neoplastikum, einem Anästhetikum und Kombinationen hiervon besteht.

10. Ein Verfahren zur Herstellung von farbkodierten polymerischen Teilchen, weil das Verfahren Folgendes umfasst:
Beschichten eines anionischen Acrylat-basierten Hydrogel-Kerns mit einer äußeren Schale;
Ausbilden einer ionischen Wechselwirkung zwischen dem Acrylat-basierten Hydrogel-Kern und einer multivalenten Metallverbindung;
wobei die äußere Schale einen oder mehrere Farbstoffe und ein Polyphosphazen umfasst, das die allgemeine Formel (I) hat: 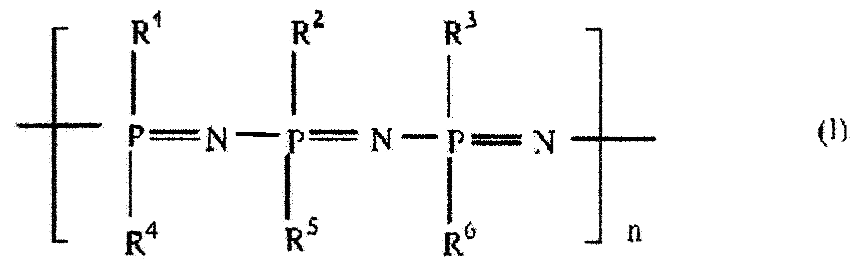
worin der n-Wert eine ganze Zahl von 2 bis ∞ ist;
R¹ bis R⁶ unabhängig aus der Gruppe ausgewählt sind, die aus:
einem substituierten oder nicht substituierten Alkyl, Aminoalkyl, Haloalkyl, Thioalkyl, Thioaryl, Alkoxy, Haloalkoxy, Aryloxy, Haloaryloxy, Alkylthiolat, Arylthiolat, Alkylsulphonyl, Alkylamino, Dialkylamino, Heterocycloalkyl mit einem oder mehreren Heteroatomen besteht, die aus Stickstoff, Sauerstoff, Schwefel, Phosphor oder einer Kombination hiervon ausgewählt sind, oder Heteroaryl, das ein oder mehrere Heteroatome umfasst, die aus Stickstoff, Sauerstoff, Schwefel, Phosphor und einer Kombination hiervon ausgewählt sind, besteht;
worin die polymerischen Teilchen als Mikrokügelchen gebildet sind, wobei jedes Mikrokügelchen mit einer kalibrierten Größe oder Größenbereich erzeugt wird, und jede Größe oder jeder Größenbereich eine optisch unterscheidbare Farbe hat, die ihm durch den einen oder mehrere Farbstoffe erteilt werden, um eine optische Anzeige der Größe oder des Größenbereiches des spezifischen Mikrokügelchen zu schaffen.

11. Das Verfahren nach Anspruch 10, bei dem R¹ bis R⁶ unabhängig aus der Gruppe ausgewählt sind, die aus OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1% oder weniger OCH₂CH=CH₂, 1% oder weniger OCH₂CH₂CH=CH₂ und irgendwelchen Kombinationen hiervon besteht.

12. Das Verfahren nach Anspruch 10, bei dem Acrylat-basierte Hydrogel-Kern ein Polymer umfasst, das aus der Gruppe ausgewählt ist, die aus Poly(MethacrylSäure, Poly(Acrylsäure), Copolymeren und Kombinationen hiervon besteht.

13. Das Verfahren nach Anspruch 10, bei dem die farbkodierten polymerischen Teilchen so kalibriert sind, dass sie einen mittleren Durchmesser aufweisen, der aus der Gruppe ausgewählt ist, die aus Folgenden besteht: von 1 µm bis 5,000 µm, von 40 µm bis 5,000 µm, von 1 bis 1,000 µm, von 200 bis 500 µm, von 1 bis 200 µm, von 40 bis 1000 µm.

14. Das Verfahren nach Anspruch 10,l bei dem die farbkodierten polymerischen Teilchen so kalibriert sind, dass sie einen mittleren Durchmesser aufweisen, der aus der Gruppe ausgewählt ist, die aus 40 µm, 100 µm, 250 µm, 400 µm, 500 µm, 700 µm, 900 µm und 1000 µm besteht.

## Revendications

1. Particules polymères chromocodées comprenant :
un noyau d'hydrogel à base d'acrylate anionique ;
un composé métallique multivalent qui forme une interaction ionique avec le noyau d'hydrogel à base d'acrylate ; et
une enveloppe externe comprenant un ou plusieurs colorants et un polyphosphazène répondant à la formule générale (I) : 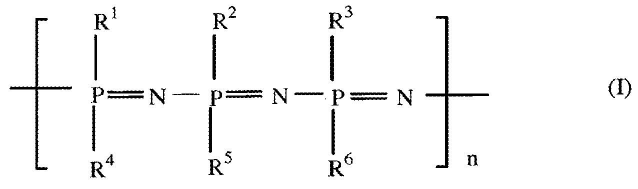
dans laquelle la valeur n est un nombre entier de 2 à ∞ ;
R¹ à R⁶ sont indépendamment choisis dans le groupe constitué par :
un groupe alkyle, aminoalkyle, halogénoalkyle, thioalkyle, thioaryle, alcoxy, halogénalcoxy, aryloxy, halogénoaryloxy, alkylthiolate, arylthiolate, alkylsulfonyle, alkylamino, dialkylamino, hétérocycloalkyle comprenant un ou plusieurs hétéroatomes choisis parmi les atomes d'azote, d'oxygène, de soufre, de phosphore, ou une combinaison de ceux-ci, ou hétéroaryle comprenant un ou plusieurs hétéroatomes choisis parmi les atomes d'azote, d'oxygène, de soufre, de phosphore, et une combinaison de ceux-ci, substitué ou non substitué
dans lesquelles les particules polymères sont formées sous forme de microsphères, chaque microsphère étant produite selon une taille ou une plage de taille et chaque taille ou plage de taille ayant une couleur visuellement distincte qui lui est donnée par un ou plusieurs colorants pour fournir une indication visuelle de la taille ou de la plage de taille de la microsphère spécifique.

2. Particules polymères chromocodées selon la revendication 1, dans lesquelles R¹ à R⁶ sont indépendamment choisis dans le groupe constitué par OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1 % ou moins de OCH₂CH=CH₂, 1 % ou moins de OCH₂CH₂CH=CH₂ et n'importe quelle combinaison de ceux-ci.

3. Particules polymères chromocodées selon la revendication 1, dans lesquelles le polyphosphazène est le poly[bis(2,2,2-trifluoroéthoxy)]phosphazène ou un dérivé du poly[bis(2,2,2-trifluoroéthoxy)]phosphazène.

4. Particules polymères chromocodées selon la revendication 1, dans lesquelles le noyau d'hydrogel à base d'acrylate comprend un polymère choisi dans le groupe constitué par un acide polyméthacrylique, un acide polyacrylique, des copolymères de ceux-ci et des combinaisons de ceux-ci.

5. Particules polymères chromocodées selon la revendication 1, calibrées pour avoir une granulométrie choisie dans le groupe constitué par : 1 µm à 5000 µm, 40 µm à 5000 µm, 1 µm à 1000 µm, 200 µm à 500 µm, 1 µm à 200 µm, 40 µm à 1000 µm.

6. Particules polymères chromocodées selon la revendication 1, calibrées pour avoir une granulométrie choisie dans le groupe constitué par 40 µm, 100 µm, 250 µm, 400 µm, 500 µm, 700 µm, 900 µm et 1000 µm.

7. Particules polymères chromocodées selon la revendication 1, qui sont bioabsorbables ou non bioabsorbables.

8. Particules polymères chromocodées selon la revendication 1, dans lesquelles le noyau comprend en outre un ou plusieurs agents actifs.

9. Particules polymères chromocodées selon la revendication 1, dans lequel le noyau d'hydrogel à base d'acrylate comprend un agent d'intérêt choisi dans le groupe constitué par le sulfate de baryum, un agent pharmaceutique, un agent de contraste, un stéroïde, une hormone, un acide nucléique, un antibiotique, un antiseptique, un analgésique, un antinéoplasique, un anesthésique et des combinaisons de ceux-ci.

10. Méthode de préparation de particules polymères chromocodées, la méthode comprenant :
le revêtement d'un noyau d'hydrogel à base d'acrylate anionique avec une enveloppe externe ;
la formation d'une interaction ionique entre le noyau d'hydrogel à base d'acrylate et un composé métallique multivalent ;
où l'enveloppe externe comprend un ou plusieurs colorants et un polyphosphazène répondant à la formule générale (I) : 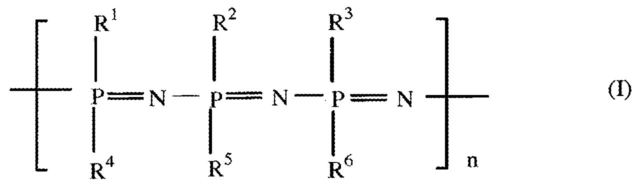
dans laquelle la valeur n est un nombre entier de 2 à ∞ ;
R¹ à R⁶ sont indépendamment choisis dans le groupe constitué par :
un groupe alkyle, aminoalkyle, halogénoalkyle, thioalkyle, thioaryle, alcoxy, halogénalcoxy, aryloxy, halogénoaryloxy, alkylthiolate, arylthiolate, alkylsulfonyle, alkylamino, dialkylamino, hétérocycloalkyle comprenant un ou plusieurs hétéroatomes choisis parmi les atomes d'azote, d'oxygène, de soufre, de phosphore, ou une combinaison de ceux-ci, ou hétéroaryle comprenant un ou plusieurs hétéroatomes choisis parmi les atomes d'azote, d'oxygène, de soufre, de phosphore, et une combinaison de ceux-ci, substitué ou non substitué,
dans laquelle les particules polymères sont formées sous forme de microsphères, chaque microsphère étant produite selon une taille ou une plage de taille et chaque taille ou plage de taille ayant une couleur visuellement distincte qui lui est donnée par un ou plusieurs colorants pour fournir une indication visuelle de la taille ou de la plage de taille de la microsphère spécifique.

11. Méthode selon la revendication 10, dans laquelle R¹ à R⁶ sont indépendamment choisis dans le groupe constitué par OCH₃, OCH₂CH₃, OCH₂CH₂CH₃, OCF₃, OCH₂CF₃, OCH₂CH₂CF₃, OCH₂CF₂CF₃, OCH(CF₃)₂, OCCH₃(CF₃)₂, OCH₂CF₂CF₂CF₃, OCH₂(CF₂)₃CF₃, OCH₂(CF₂)₄CF₃, OCH₂(CF₂)₅CF₃, OCH₂(CF₂)₆CF₃, OCH₂(CF₂)₇CF₃, OCH₂CF₂CHF₂, OCH₂CF₂CF₂CHF₂, OCH₂(CF₂)₃CHF₂, OCH₂(CF₂)₄CHF₂, OCH₂(CF₂)₅CHF₂, OCH₂(CF₂)₆CHF₂, OCH₂(CF₂)₇CHF₂, 1 % ou moins de OCH₂CH=CH₂, 1 % ou moins de OCH₂CH₂CH=CH₂ et n'importe quelle combinaison de ceux-ci.

12. Méthode selon la revendication 10, dans laquelle le noyau d'hydrogel à base d'acrylate comprend un polymère choisi dans le groupe constitué par un acide polyméthacrylique, un acide polyacrylique, des copolymères de ceux-ci et des combinaisons de ceux-ci.

13. Méthode selon la revendication 10, dans laquelle les particules polymères chromocodées sont calibrées pour avoir une granulométrie choisie dans le groupe constitué par : 1 µm à 5000 µm, 40 µm à 5000 µm, 1 µm à 1000 µm, 200 µm à 500 µm, 1 µm à 200 µm, 40 µm à 1000 µm.

14. Méthode selon la revendication 10, dans laquelle les particules polymères chromocodées sont calibrées pour avoir une granulométrie choisie dans le groupe constitué par 40 µm, 100 µm, 250 µm, 400 µm, 500 µm, 700 µm, 900 µm et 1000 µm.
